# DEMANDE DE BREVET EUROPEEN

(11) **EP 0 552 995 A1**
(43) Date de publication de la demande: **28.07.1993**
(21) Numéro de dépôt: 93400013.4
(22) Date de dépôt: 05.01.1993
(51) Int. Cl.: A61F 7/00, A61B 17/22

(54) **Procédé de visée d'une cible anatomique en vue de son traitement par ondes élastiques focalisées et appareil faisant application de ce procédé au traitement de la prostate par hyperthermie**

(30) Priorité: 21.01.1992 FR 9200577
(71) Demandeur: EDAP INTERNATIONAL, F-77200 Croissy Beaubourg-Marne la Vallée (FR)
(72) Inventeur: Dory, Jacques, F-77450 Coupvray (FR)
(74) Mandataire: Hirsch, Marc-Roger

(57) **Abrégé**

Le générateur de traitement est déplaçable suivant trois axes de coordonnées (XYZ) et une sonde endorectale (8) suivant trois axes parallèles à (XYZ) et liés au support (7) du patient. Un premier repère (A), lié à la sonde, est situé dans le même plan horizontal que son foyer et décalé d'une distance (d) par rapport à lui dans la direction de l'axe (Y). Un second repère (B), lié au dispositif de déplacement du générateur de traitement, est situé dans le plan horizontal du foyer de ce dernier et décalé par rapport à lui de la même distance (d) dans la même direction.

## Description

L'invention concerne un perfectionnement aux appareils de traitement, en particulier par hyperthermie, du type comportant un générateur d'un faisceau focalisé d'ultrasons de traitement, associé à une sonde échographique dont le plan de balayage passe par le point focal du faisceau de traitement, à des moyens de déplacement et d'orientation du générateur de traitement et de la sonde suivant des axes de coordonnées et à un dispositif de formation et de visualisation d'une image échographique de la cible, comportant des moyens de matérialisation visuelle de la position théorique dudit point focal, en vue de permettre la mise en coïncidence de ladite position avec un point de l'image de la cible.

Un tel appareil fait notamment l'objet du brevet français No 84 06877, déposé le 3 mai 1984, pour : "Appareil d'examen et de localisation de tumeurs par ultrasons muni d'un dispositif de traitement localisé par hyperthermie", suivant lequel la sonde est, de préférence, disposée dans l'axe du transducteur de traitement, et solidarisée à ce dernier.

Dans le traitement de la prostate par exemple, l'image échographique fournie par la sonde axiale n'est pas toujours satisfaisante.

Il est connu en diagnostic de procéder à l'examen échographique de cet organe au moyen d'une sonde endorectale, qui fournit des images excellentes.

L'invention a pour premier objet d'associer une sonde échographique endorectale à un appareil du type susvisé, agencé pour que les moyens de déplacement assurent la mise en coïncidence de la position théorique du point focal du faisceau de traitement avec un point prédéterminé de l'image de la cible fournie par la sonde endorectale, en vue d'utiliser cette dernière pour effectuer la visée.

Elle a pour objet plus général un procédé de visée d'une cible anatomique en vue de son traitement par un faisceau focalisé d'ondes élastiques de traitement, comportant la génération d'un faisceau focalisé d'examen par une sonde échographique à temps réel, les déplacements du générateur dudit faisceau de traitement et de ladite sonde en liaison avec un système de coordonnées, et la formation et la visualisation d'une image échographique de la cible, ainsi que la matérialisation par un repère visuel de la position théorique d'un point repère situé dans l'un des deux faisceaux, en vue de permettre la visée par mise en coïncidence de ladite position avec un point de l'image de la cible, caractérisé par le fait que ledit repère visuel R matérialise la position du point focal du faisceau d'examen, par la mise en place d'un premier et d'un second repères dont les positions dans l'espace sont respectivement définies par rapport au support fixe du corps du patient et au support mobile du générateur de traitement et liées en permanence aux positions respectives du foyer F de traitement et du repère R, lesdites positions se déduisant respectivement de celles de F et de R par un déplacement prédéterminé par rapport au système de coordonnées, et par le fait que la mise en coïncidence du foyer F du faisceau de traitement avec la cible s'obtient par la mise en coïncidence de l'image échographique avec ledit repère visuel, svivie du déplacement du générateur de traitement jusqu'à mise en coïncidence du premier et du second repères.

Suivant un mode d'exécution préféré, un appareil de traitement de la prostate, dans lequel lesdits moyens de déplacement du générateur de traitement comportent un arceau supporté par l'intermédiaire d'une pièce horizontale autour de laquelle il peut être entraîné en rotation, autour de son axe horizontal, par une pièce verticale elle-même déplaçable suivant trois axes de coordonnées rectangulaires XYZ, ledit axe horizontal étant parallèle à l'axe Y, et des moyens d'autoriser la rotation de l'arceau autour d'un axe parallèle à l'axe X et qui coupe ledit axe horizontal parallèle à l'axe Y au foyer du générateur de traitement, le support plan du patient étant parallèle au plan XY, est caractérisé par une sonde endorectale déplaçable suivant trois axes de coordonnées rectangulaires parallèles aux axes XYZ, liés audit support plan, et orientable dans une direction quelconque de l'espace, le premier repère, lié à la sonde, étant situé dans le même plan horizontal que le foyer de la sonde et décalé par rapport à lui dans la direction de l'axe Y d'une distance prédéterminée, tandis que le second repère, lié à la pièce de support de l'arceau, est situé dans la plan horizontal du foyer du générateur de traitement et décalé par rapport à lui de ladite distance prédéterminée dans la direction de l'axe Y.

D'autres particularités, ainsi que les avantages de l'invention, apparaîtront clairement à la lumière de la description ci-après.

Au dessin annexé :

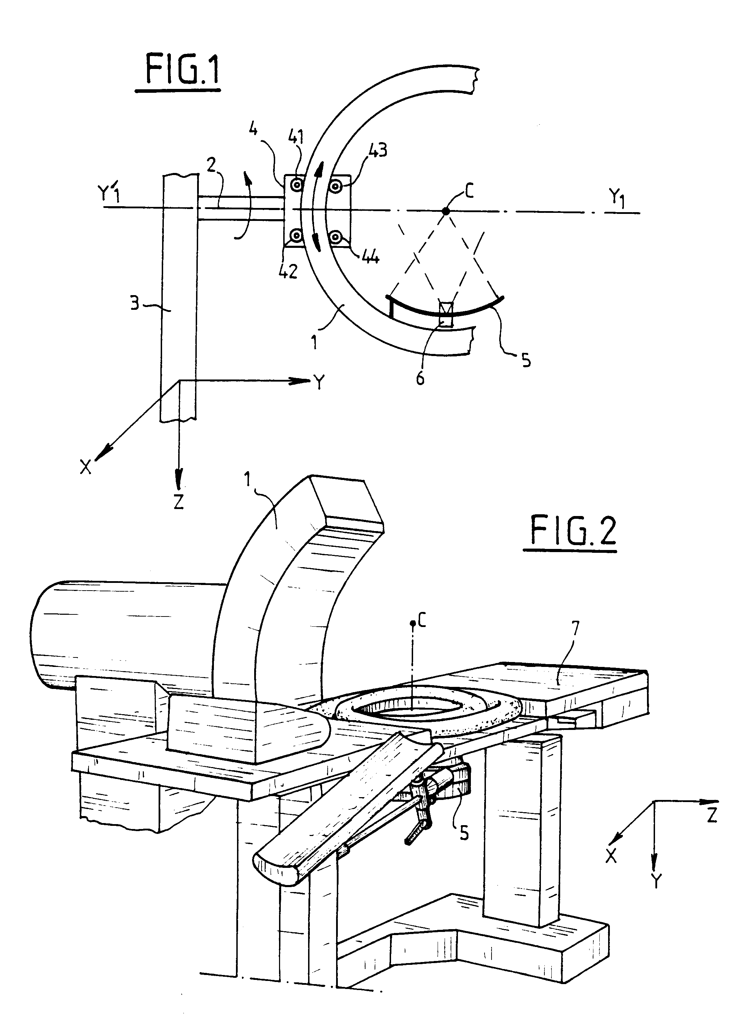

La figure 1 représente schématiquement, vus de profil, l'arceau d'un appareil d'hyperthermie conforme à un mode d'exécution préféré de l'invention, ainsi que la tête de traitement et la sonde échographique axiale ;
La figure 2 est une vue partielle en perspective de mêmes organes et de la table de traitement ; et
Les figures 3 et 4 illustrent de façon symbolique, respectivement en plan et en élévation, le positionnement de la sonde endorectale par rapport à la sonde de support du patient.

On voit au dessin que l'arceau 1 est supporté, par l'intermédiaire d'une pièce horizontale 2 autour de laquelle il peut être entraîné, par des moyens non figurés, en rotation autour de son axe horizontal Y'₁Y₁, par une pièce verticale 3 elle-même déplaçable suivant trois axes de coordonnées rectangulaires XYZ, Y'₁Y₁ étant parallèle à l'axe Y.

Par ailleurs, un chariot 4 muni de galets 41-44 autorise un mouvement de rotation de l'arceau 1 autour d'un axe parallèle à l'axe X et qui coupe l'axe Y'₁Y₁ en un point C.

Les cinq déplacements autorisés de l'arceau 1 sont provoqués par des moteurs eux-mêmes commandés par exemple par des manches à balai.

Un tel dispositif de commande des déplacements est connu en soi et ne sera pas décrit plus en détail.

Le transducteur de traitement de l'appareil a été symbolisé sous la forme d'une coupelle sphérique 5 fixée à la partie inférieure de l'arceau 1 et dont le foyer est en C, quelles que soient la position et l'orientation de l'arceau.

La sonde échographique axiale 6 est disposée suivant l'axe de la coupelle 5 et solidaire de celle-ci.

L'appareil est du type décrit dans le brevet susvisé. L'arceau 1 permet, (une fois que des déplacements en XYZ lui ont été communiqués afin d'amener le foyer du transducteur 5 en coïncidence avec un point de la cible, de la manière expliquée dans ledit brevet), d'orienter la tête de traitement et d'examen échographique 5-6 autour de deux axes respectivement parallèles aux axes X et Y.

Cette orientation facilite la recherche d'une fenêtre acoustique, en fonction du patient et de la position de la cible dans son corps.

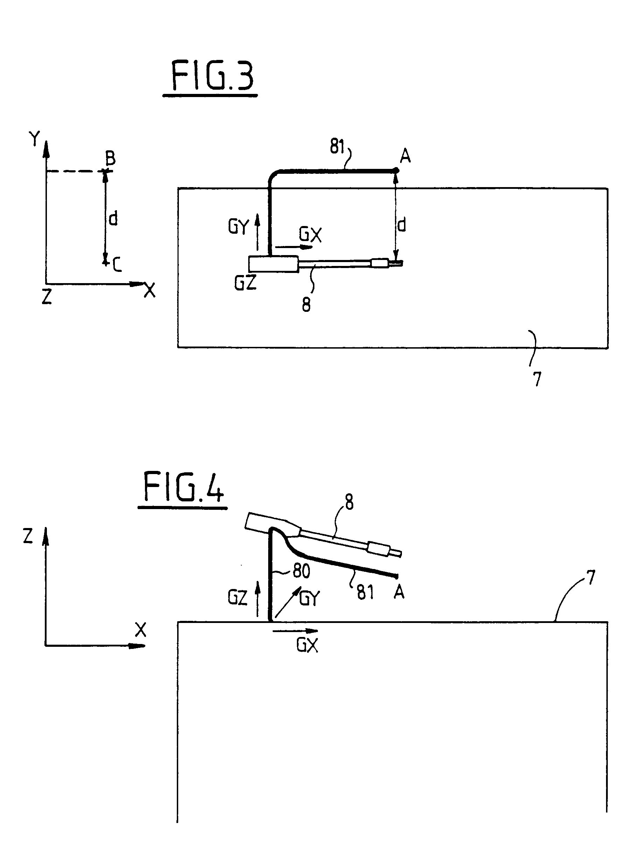

Aux figures 3 et 4, on a représenté symboliquement le positionnement de la sonde endorectale 8 par rapport à la table 7 sur laquelle le patient est allongé.

La sonde 8 est montée à l'extrémité d'un support 80 déplaçable par rapport à la table suivant trois axes de coordonnées au moyen de trois glissières symbolisées par des flèches GX, GY, GZ et dont la position peut être verrouillée une fois réglée. Son plan de balayage est par exemple perpendiculaire à l'axe longitudinal de son corps, mais il pourrait aussi lui être parallèle.

Le montage permet également l'orientation de la sonde dans une direction quelconque.

Un repère A est matérialisé par l'extrémité d'une tige 81 solidaire de la sonde 8.

Ce repère est situé dans le même plan horizontal que le foyer f de ladite sonde, mais décalé par rapport à lui d'une distance d prédéterminée suivant l'axe des Y. La distance d est par exemple fixée à une valeur comprise entre 10 et 15 cm.

La sonde 8 est reliée de la manière classique au dispositif échographique de l'appareil, sur l'écran duquel on a matérialisé un repère fixe fi dont la position a été déterminée en utilisant une petite cible métallique auxiliaire, positionnée de telle sorte par rapport à la sonde 8 que l'on obtienne une image de cette cible sur l'écran, en mémorisant les coordonnées de l'image et en affichant électroniquement une croix à la position définie par ces coordonnées.

A partir des coordonnées connues de cette cible auxiliaire, par décalage de 15 cm, par exemple, dans un sens prédéterminé, ici suivant l'axe des Y, on définit la position du repère A (on met en place la tige). Après ce réglage préalable, fait une fois pour toutes, de l'appareil, la cible auxiliaire est enlevée.

Un repère B, également matérialisé par l'extrémité d'une tige solidaire du support de l'arceau, est situé dans le plan horizontal du foyer C du transducteur de traitement et décalé par rapport à C de la distance d définie ci-dessus, suivant l'axe des Y et dans le même sens que le repère A.

Il doit être bien compris que les repères A et B pourraient être matérialisés par tout autre moyen, mécanique ou optique par exemple.

Pour obtenir le positionnement des sondes nécessaire à la localisation de la cible anatomique et à sa visée par le transducteur de traitement, on opère alors de la manière suivante :

La sonde endorectale est convenablement orientée et mise en place dans le corps du patient en vue d'obtenir une image de la cible anatomique coïncidant avec la croix visualisée en permanence sur l'écran, ce qui est possible, du fait que la cible auxiliaire avait été positionnée dans une zone voisine de celle qu'occupera le foyer f pendant les traitements, et du fait que l'on dispose d'une certaine latitude de mise en place de la sonde par rapport au patient pour obtenir que la position relative de la cible en XYZ par rapport à la sonde reste la même que lors du réglage préalable.

Le repère A étant lié à la sonde dans ses déplacements, il reste évidemment décalé de 15 cm en suivant l'axe des Y par rapport au foyer f de la sonde, quelle que soit la position de celui-ci.

On déplace alors l'arceau pour amener le repère B en coïncidence dans l'espace avec le repère A : il en résulte que le foyer du transducteur de traitement aura ainsi été mis en coïncidence avec la position occupée par le foyer de la sonde, donc avec un point de la cible.

On oriente ensuite l'arceau de façon à tenter d'obtenir une image échographique correcte au moyen de la sonde axiale 6.

Celle-ci sert à confirmer l'examen fait avec la sonde endorectale, mais n'est pas toujours en mesure de donner une image satisfaisante, et n'est pas rigoureusement indispensable, la sonde endorectale suffisant en principe à la localisation de la cible, à la visée et au contrôle échographique du traitement.

Il doit être bien compris que le procédé décrit de visée d'une cible en vue de son traitement par un faisceau focalisé d'ondes élastiques pourrait être appliqué avec une sonde destinée à l'examen d'une partie quelconque du corps du patient.

Il consiste en effet, de manière générale, à déplacer la sonde par rapport à un système de coordonnées, avantageusement rectangulaires, lié au plan de support du patient - le générateur du faisceau focalisé de traitement étant lui-même déplacé par rapport à un système de coordonnées indépendant du précédent, mais avantageusement parallèle à lui - à définir par un réglage préalable une position fixe d'un repère visuel permanent sur l'écran échographique, ainsi qu'un premier repère matériel lié à la sonde de manière constante dans ses déplacements, avantageusement situé dans le plan passant par le foyer de la sonde et parallèle audit plan de support et décalé par rapport audit foyer d'une distance prédéterminée dans une direction et un sens prédéterminés, à lier au générateur de traitement un second repère matériel, à former, au moyen de ladite sonde, une image échographique de la cible à traiter qui coïncide sur l'écran avec le repère visuel et à déplacer le générateur de traitement pour amener le second repère en coïncidence avec le premier.

D'une façon plus générale, le système de coordonnées n'étant pas nécessairement rectangulaire, il reste que les deux repères matériels, dont les positions sont respectivement définies par rapport au support fixe du patient et au support mobile du générateur de traitement, sont liés en permanence aux foyers des deux générateurs de faisceaux focalisés respectifs par la même relation, (leur position se déduit de celles du foyer correspondant par une translation ou, plus généralement, par un autre déplacement prédéterminé) et la mise en coïncidence du foyer du générateur de traitement avec la cible s'obtient par la mise en coïncidence de l'image échographique avec un repère visuel prédéterminé sur l'écran, suivie du déplacement du générateur de traitement pour obtenir la coïncidence des deux repères matériels.

Ce procédé permet d'effectuer de manière très simple une visée de grande précision, résultat très difficile à atteindre au moyen des procédés de localisation faisant appel à un bras de support de la sonde asservi en position de manière aveugle au moyen d'un système informatique.

## Revendications

1. Procédé de visée d'une cible anatomique en vue de son traitement par un faisceau focalisé d'ondes élastiques de traitement, comportant la génération d'un faisceau focalisé d'examen par une sonde échographique à temps réel, les déplacements du générateur dudit faisceau de traitement et de ladite sonde en liaison avec un système de coordonnées, et la formation et la visualisation d'une image échographique de la cible, ainsi que la matérialisation par un repère visuel de la position théorique d'un point repère situé dans l'un des deux faisceaux, en vue de permettre la visée par mise en coïncidence de ladite position avec un point de l'image de la cible,
caractérisé par le fait que ledit repère visuel (R) matérialise la position du point focal du faisceau d'examen, par la mise en place d'un premier et d'un second repères dont les positions dans l'espace sont respectivement définies par rapport au support fixe du corps du patient et au support mobile du générateur de traitement et liées en permanence aux positions respectives du foyer (F) de traitement et du repère (R), lesdites positions se déduisant respectivement de celles de (F) et de (R) par un déplacement prédéterminé par rapport au système de coordonnées, et par le fait que la mise en coïncidence du foyer (F) du faisceau de traitement avec la cible s'obtient par la mise en coïncidence de l'image échographique avec ledit repère visuel, suivie du déplacement du générateur de traitement jusqu'à mise en coïncidence du premier et du second repères.

2. Procédé selon la revendication 1,
caractérisé en ce que les déplacements de la sonde et du générateur de traitement sont respectivement définis par rapport à un système de coordonnées rectangulaires lié au support plan du patient et à un système de coordonnées rectangulaires parallèle au précédent et que ladite translation est parallèle audit support plan, et transversale par rapport au corps du patient.

3. Procédé suivant la revendication 1 ou 2,
caractérisé en ce que la mise en place du premier repère lié au foyer du faisceau d'examen et du repère visuel sur l'écran échographique s'effectuent une fois pour toutes au moyen d'une cible auxiliaire dont la position est connue par rapport à la sonde d'examen et dont on forme l'image échographique sur l'écran pour déterminer l'emplacement du repère visuel, la position du premier repère se déduisant de celle de la cible auxiliaire par ledit déplacement, et la sonde échographique étant, avant chaque traitement, déplacée par rapport au patient jusqu'à ce que l'image échographique de la cible anatomique coïncide avec ledit repère visuel.

4. Procédé selon l'une des revendications 1 à 3,
caractérisé en ce que lesdits premier et second repères sont constitués par les extrémités de pièces métalliques allongées respectivement liées à la sonde échographique et au dispositif de déplacement du générateur de traitement.

5. Appareil de traitement de la prostate faisant application du procédé selon l'une des revendications 1 à 4, dans lequel lesdits moyens de déplacement du générateur de traitement (5) comportent un arceau (1) supporté, par l'intermédiaire d'une pièce horizontale (2) autour de laquelle il peut être entraîné en rotation autour de son axe horizontal, par une pièce verticale (3) elle-même déplaçable suivant trois axes de coordonnées rectangulaires (XYZ), ledit axe horizontal (Y'₁Y₁) étant parallèle à l'axe (Y), et des moyens (4-41-42) d'autoriser la rotation de l'arceau autour d'un axe parallèle à l'axe (X) et qui coupe l'axe (Y'₁Y₁) au foyer (C) du générateur de traitement, le support plan (7) du patient étant parallèle au plan (XY),
caractérisé par une sonde endorectale déplaçable suivant trois axes de coordonnées rectangulaires parallèles aux axes (XYZ), liés audit support plan, et orientable dans une direction quelconque de l'espace, le premier repère (A), lié à la sonde, étant situé dans le même plan horizontal que le foyer de la sonde et décalé par rapport à lui dans la direction de l'axe (Y) d'une distance prédéterminée, tandis que le second repère (B), lié à la pièce (3) de support de l'arceau, est situé dans la plan horizontal du foyer du générateur de traitement et décalé par rapport à lui de ladite distance prédéterminée dans la direction de l'axe (Y).

6. Appareil selon la revendication 5,
caractérisé en ce qu'il comporte en outre une deuxième sonde échographique à temps réel (6) liée à la surface de focalisation (5) du faisceau du générateur de traitement et dont le plan de balayage passe par le foyer (C) de celui-ci.
